# EUROPÄISCHE PATENTANMELDUNG

(11) **EP 2 246 326 A1**
(43) Veröffentlichungstag der Anmeldung: **03.11.2010**
(21) Anmeldenummer: 09006050.0
(22) Anmeldetag: 02.05.2009
(51) Int. Cl.: C07C 319/20, C07C 323/58, C07D 209/30, C07D 209/34

(54) **Verfahren zur Herstellung von Oxindolen und ortho-substituierten Anilinen und ihre Verwendung als Zwischenprodukte für Synthesen**

(71) Anmelder: Bayer CropScience AG, 40789 Monheim (DE)
(72) Erfinder: Ford, Mark, James, Dr., 61389 Schmitten (DE); Karig, Gunter, Dr., 65719 Hofenheim (DE)

(57) **Zusammenfassung**

Verfahren zur Herstellung von Oxindolen und ortho-substituierten Anilinen und ihre Verwendung als Zwischenprodukte für Synthesen
Verfahren zur Herstellung von Verbindungen der Formel (4): 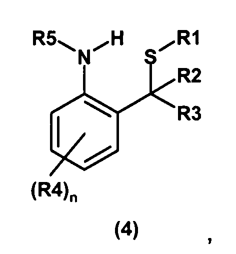 wobei man eine Mischung eines Anilins (Verbindung der Formel Q) mit einem Thioether (Verbindung der Formel W): 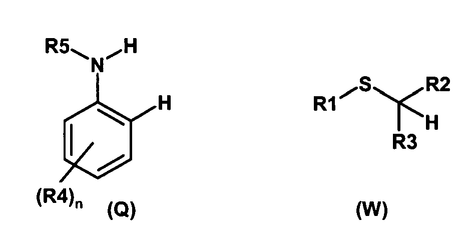 in Gegenwart eines Chlorierungsmittels und eines organischen Lösungsmittels bei einer Reaktionstemperatur im Bereich von über -65°C umsetzt.
In einem Folgeverfahren wird diese Verbindung in Gegenwart eines Säure- Katalysators zu dem Indol der Formel (7) oder dem Oxindol der Formel (8) weiter umgesetzt:

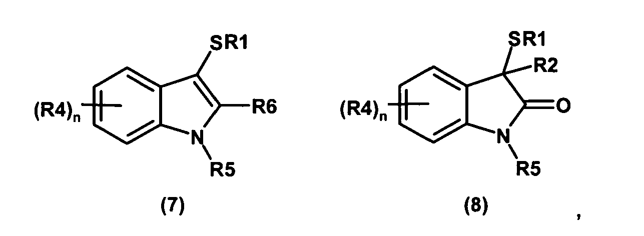

## Beschreibung

Die Erfindung betrifft das technische Gebiet der chemischen Synthese von biologisch aktiven Verbindungen, vorzugsweise für Zwischenprodukte für die Synthese von Feinchemikalien und Wirkstoffen aus der Pharmazie und/oder der Landwirtschaft.

Prinzipiell gehört der selektive Austausch von Wasserstoff an einem aromatischen System mit einem substituierten Kohlenstoff-Atom zu einer der fundamentalen Umsetzungen in der organischen Chemie und ist damit bekannt.

Eine Klasse von Verbindungen, die so hergestellt werden können, sind 2-Oxindole (Dihydroindol-2-one) und deren Vorprodukte. Oxindole und deren Vorprodukte sind vielseitig verwendbare Zwischenprodukte für Wirkstoffsynthesen (Bioorg. Med. Chem. Lett. 2006, 16, 2109; JP 2008-101014; WO 96/41799 A1).

Die meisten der beschriebenen, unterschiedlichen Synthesen von Oxindolen verwenden eine Variation einer Friedel-Crafts-Reaktion (Stolle Synthesis, W.C. Sumpter, Chem. Rev. 1945, 37, 443-449). Jedoch sind Stolle-Synthesen nur bedingt einsetzbar, da sie stark saure Bedingungen und ein elektronenreiches Anilin benötigen. Daneben sind aber auch Radikal-, Nitrenium-ion- und Organolithium-Reaktionen sowie photochemisch abhängige Methoden bekannt. Diese sind aber auch limitiert durch den herzustellenden Typ von Oxindolen, die Kompatibilität von Substraten, den Reaktionsbedingungen sowie durch den Umstand, dass der Aromat schon einen Halogen-Substituenten besitzen muss, welcher dann ersetzt wird. (Radikal-Verfahren: Zard et al., Tetrahedron Lett. 1994, 35, 9553-9556; Zard et al., Tetrahedron Lett. 1994, 35, 1719-1722; Jones et al., Tetrahedron Lett. 1994, 35, 7673-7676; Kikugawa et al., Chem. Letters 1987, 1771-1774; Clark et al., Synthesis 1991, 871-878; Yonemitsu et. al., Chem. Pharm. Bull. 1981, 29, 128-136; s. Schema 1).

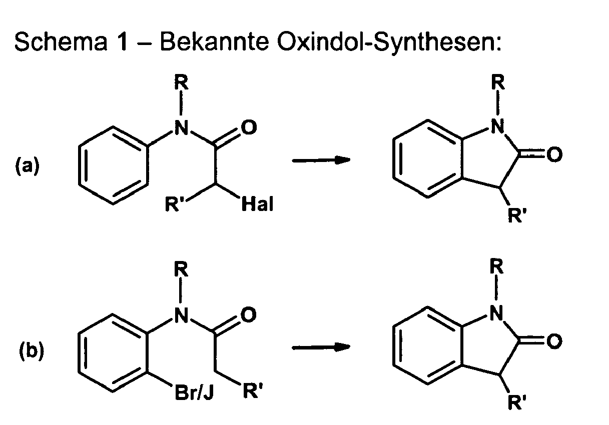

Das Verfahren von Gassman et al. (Organic Synthesis Coll., Vol. 6, 601 und Vol. 56, 72), welches von Anilin und Methylthioacetat-Ester über Chlorierung und Behandlung mit Triethylamin bei -70°C abläuft, erscheint geeignet in Sinne von Durchführbarkeit, Verfügbarkeit von Edukten, kurzer Reaktionsgeschwindigkeit und Reproduzierbarkeit. Jedoch wird aber auch beschrieben, dass gute Ausbeuten nur erzielt werden können, wenn die instabilen N-Chlor- (1) oder N-Sulfonium- (2) Zwischenprodukte unter -65°C, im Normalfall bei -78°C gebildet werden (Gassman et. al., J. Am. Chem. Soc., 1974, 96(17), 5508; Gassman et al., J. Am. Chem. Soc., 1974, 96(17), 5512; WO 96/41799 A1; s. Schema 2).

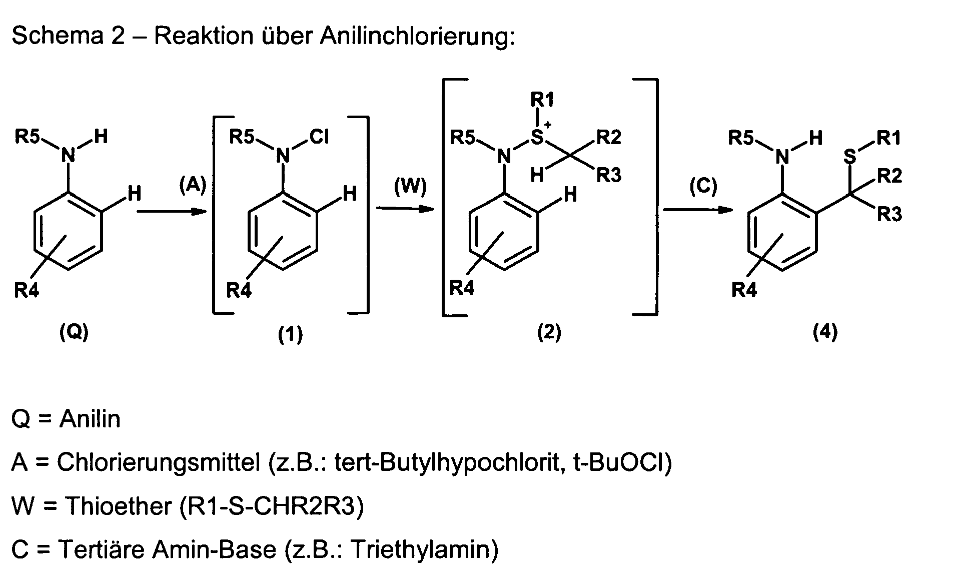

Das Chlorierungsmittel der Wahl gemäß Literatur ist das instabile und explosive tert-Butylhypochlorit, da das Nebenprodukt der Chlorierung dann den neutralen tert-Butylalkohol ergibt. In den wenigen Fällen, worin Sulfurylchlorid (SO₂Cl₂) eingesetzt worden war, wurde eine zweite, nicht nukleophile Base, wie "proton sponge", eingesetzt (Johnson, J. Org. Chem. 1990, 55, 1374; Warpehoski, Tetrahedron Lett. 1986, 27, 4103). Da beide Varianten bei tiefen Temperaturen durchgeführt werden, ist dies jedoch keine gangbare Lösung im technisch Maßstab.

Wright et al. (Tetrahedron Lett. 1996, 37, 4631) beschreibt eine Alternative, wobei das Chlorsulfonium-Zwischenprodukt (3) aus einem Sulfoxid und Oxalylchlorid hergestellt wurde (s. Schema 3). Hierbei ist das Chlorsulfonium-Zwischenprodukt (3) ebenfalls instabil. Für diese Reaktion muss das Sulfoxid zuerst hergestellt und isoliert werden. Aus Gründen der Stabilität muss die Reaktion bei -78°C ablaufen und um eine Reaktion zwischen Anilin und Oxalylchlorid zu vermeiden, wird die Reaktion stufenweise durchgeführt.

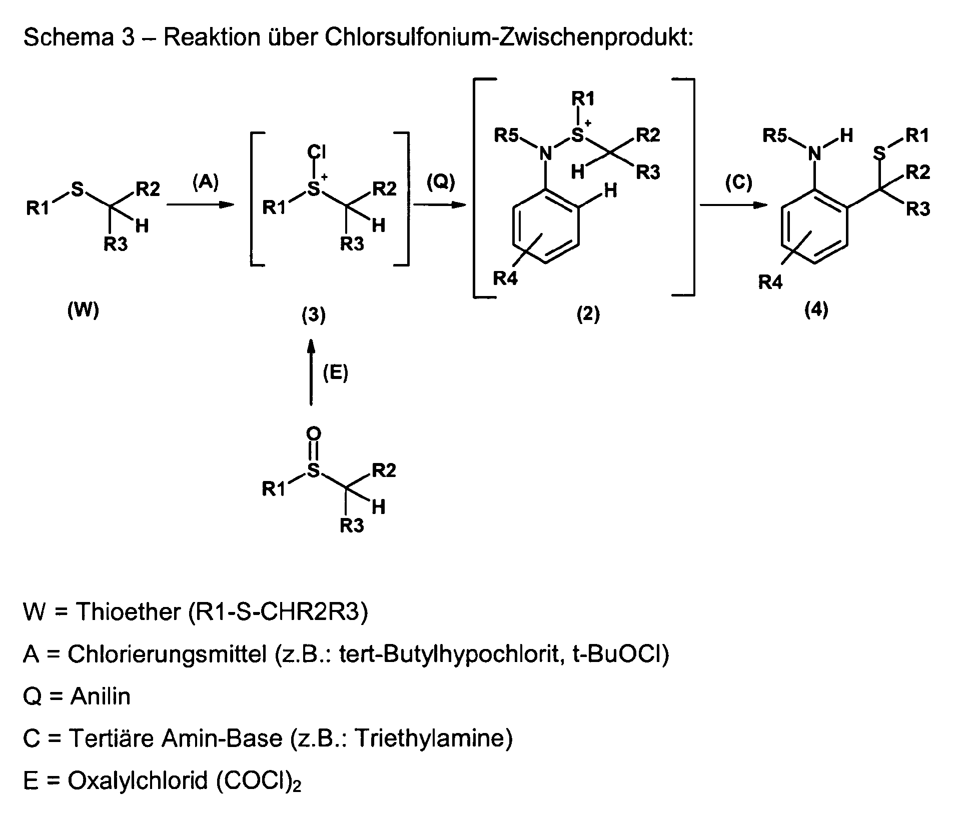

Die Gründe, warum die Reaktion so empfindlich gegenüber Reaktionstemperaturen über -70°C ist und warum es immer stufenweise durchgeführt wurde, sind vielfältig.

Zuerst einmal kommen die funktionellen Gruppen, die an der Reaktion teilnehmen, d.h. das Stickstoff-Atom des Anilins und das Schwefel-Atom des Thioethers, unverändert sowohl im Produkt (4) als auch im Edukt vor. Somit wäre eine selektive Chlorierung während der Reaktion nicht zu erwarten, in der das Produkt (4) gleich gebildet wird. Aus diesem Grunde verwenden alle literaturbekannten Methoden eine stufenweise Reaktion.

Daneben kann sich bei höheren Temperaturen als -65°C das N-Chloranilin in einen im Kern chlorierten Aromaten umwandeln, wie auch andere Oxidationsprodukte (Dimere) bilden. Somit ist es nicht überraschend, dass das deutlich weniger elektronenreiche, und somit deutlich weniger zur Kern-Chlorierung-reaktive Acetanilid nur Kern-Chlorierungen mit tert-Butylhypochlorit bei 0°C bildet (Lengyel et. al. Synth. Comm., 1998, 28 (10), 1891-1896).

Weiterhin können die Sulfonium-Zwischenprodukte (2) oder (3) in Gegenwart von Basen eliminieren und das reaktive Nebenprodukt (5) bilden, das z.B. mit einem Anilin kondensieren würde, wodurch die Nebenkomponente (6) irreversibel erzeugt wird (s. Schema 4). Dies entspricht der sog. Pummerer-Oxidation des R2-CH-R3-Restes.

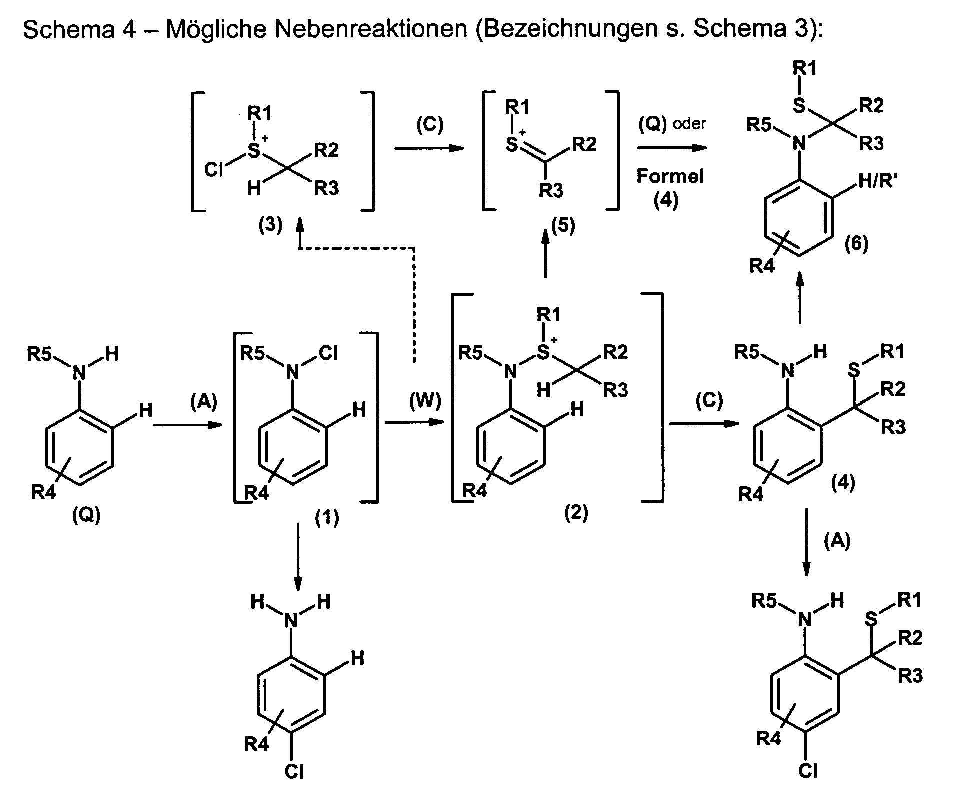

Es bestand daher die Aufgabe, ein modifiziertes Verfahren bereitzustellen, das eine im Vergleich zu den obengenannten Verfahren verbesserte bzw. realisierbare Herstellung im technischen Maßstab von Zwischenprodukten (4) bedeutet und vorzugsweise auch eine Weiterverarbeitung zu Oxindolen mit Vorteilen, wie verbesserter Gesamtausbeute und/oder Produktreinheit, verringertem Einsatz von Ausgangsmaterialien oder vereinfachtem Verfahrensablauf erlaubt, wie z.B. Reaktionen bei höherer Temperatur.

Überraschend wurde nun gefunden, dass trotz der oben genannten Probleme die Reaktion so modifiziert werden konnte, dass die Reaktion in einem industriellrealisierbaren Maßstab durchgeführt werden kann.

Gegenstand der Erfindung ist ein Verfahren zur Herstellung von Verbindungen der Formel (4): 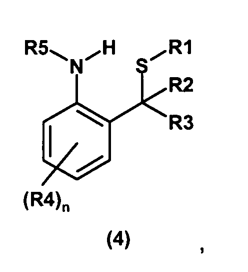 worin jeder der Reste
- R1: = C1-C6 Alkyl, substituiertes Alkyl, Aryl oder substituiertes Aryl, vorzugsweise C1-C4 Alkyl;
- R2: = H, C1-C6 Alkyl, substituiertes Alkyl, Aryl oder substituiertes Aryl;
- R3: = elektronenziehende oder aktivierende Gruppe, wie -CO-R1; -CO-X, wobei X = OR1, SR1, NR2R2', wobei R2' wie R2 definiert ist und gleich oder anders als R2 sein kann; R2 und R2' können einen Ring bilden; SO(n')-R1, wobei n' ist 0, 1, or 2; -CN; -NO₂, Aryl or Heteroaryl;
- R4: = F, Cl, Br, I, CF₃, CN, NO₂, COX; wobei X = OR1, SR1, NR2R2', wobei R2' wie R2 oben definiert ist und gleich oder anders als R2 sein kann, vorzugsweise F, insbesondere 2-F;
- n: = 1 - 4, vorzugsweise 1 - 2 , insbesondere 1;
- R5: = H, C1-C6 Alkyl, substituiertes Alkyl, Aryl oder substituiertes Aryl; bedeutet,
**dadurch gekennzeichnet, dass** man eine Mischung
eines Anilins (Verbindung der Formel Q): 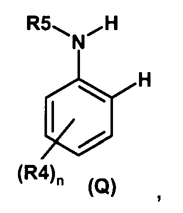 worin die Reste R4, n und R5 wie in Formel (4) definiert sind,
mit einem Thioether (Verbindung der Formel W): 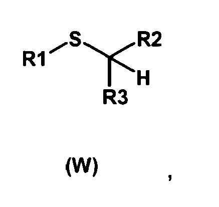 worin die Reste R1, R2 und R3 wie in Formel (4) definiert sind,
in Gegenwart eines Chlorierungsmittels und eines organischen Lösungsmittels bei einer Reaktionstemperatur im Bereich von über -65°C, vorzugsweise zwischen -60 und -10°C, insbesondere zwischen -50 und -20°C, zur Verbindung der Formel (4) umsetzt.

Die hieraus resultierende selektive und saubere Reaktion mit guten Ausbeuten ist besonders überraschend und steht im Gegensatz zur Lehrmeinung, wonach beschrieben wird, dass gute Ausbeuten nur erzielt werden können, wenn die instabilen N-Chlor- (1) oder N-Sulfonium- (2) Zwischenprodukte (s. Schema 2) unter -65°C, im Normalfall bei -78°C gebildet werden (Gassman et. al., J. Am. Chem. Soc., 1974, 96(17), 5508; Gassman et al., J. Am. Chem. Soc., 1974, 96(17), 5512; WO 96/41799 A1).

Der im erfindungsgemäßen Verfahren verwendete Thioether (Verbindung der Formel W) hat die folgende Struktur: 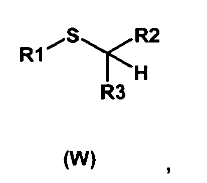 wobei
R1 = C1-C6 Alkyl, substituiertes Alkyl, Aryl oder substituiertes Aryl, vorzugsweise C1-C4 Alkyl;
R2 = H, C1-C6 Alkyl, substituiertes Alkyl, Aryl oder substituiertes Aryl;
R3 = elektronenziehende oder aktivierende Gruppe, wie -CO-R1; -CO-X,
wobei X = OR1, SR1, NR2R2', wobei R2' wie R2 definiert ist und gleich oder anders als R2 sein kann; R2 und R2' können einen Ring bilden;
SO(n')-R1, wobei n' ist 0, 1, or 2; -CN; -NO₂, Aryl or Heteroaryl;
bedeutet.
Soweit im Folgenden auf Thioether verwiesen wird, ist die oben genannte Verbindung gemeint.

Das im erfindungsgemäßen Verfahren verwendete Anilin (Verbindung der Formel Q) hat die folgende Struktur: 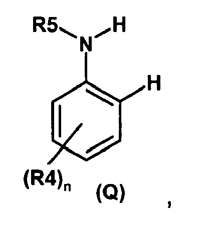 wobei
- R4: = F, Cl, Br, I, CF₃, CN, NO₂, COX; wobei X = OR1, SR1, NR2R2', wobei R2' wie R2 oben definiert ist und gleich oder anders als R2 sein kann, vorzugsweise F, insbesondere 2-F;
- n: = 1 - 4, vorzugsweise 1 - 2 , insbesondere 1;
- R5: = H, C1-C6 Alkyl, substituiertes Alkyl, Aryl oder substituiertes Aryl; bedeutet.

Zwingend ist, dass ein Wasserstoffatom in ortho-Position zum Stickstoffatom des Anilins vorliegt. Der Ring kann mit einem oder mehreren Substituenten R4 substituiert sein. Soweit im Folgenden auf Anilin verwiesen wird, ist die oben genannte Verbindung gemeint.

Als Chlorierungsmittel kommen alle dem Fachmann hierfür bekannten Chlorierungsmittel in Frage, wie N-Chlorsuccinimid, Trichlorisocyanursäure tert-Butylhypochlorit und Sulfyrylchlorid. Bevorzugt sind Chlorierungsmittel wie Sulfurylchlorid (SO₂Cl₂), welche überraschend gegenüber der Literaturmeinung HCl generieren, ohne dass eine zweite, nicht nucleophile Base eingesetzt werden muss.

Die Reaktion im erfindungsgemäßen Verfahren kann mit verschiedenen Lösungsmitteln durchgeführt werden.

So können unpolare organische Lösungsmittel eingesetzt werden, wie Chloralkane (beispielsweise Dichlormethan und Dichlorethan), Aromaten (beispielsweise Benzol, Toluol, Xylol), Haloaromaten (beispielsweise Chlorbenzol, Dichlorbenzol), substituierte Aromaten (beispielsweise Benzotriflurid, Chlorbenzotrifluorid, Chlortoluol, Chlorxylol) allein oder als Mischungen - oder als Mischungen mit Alkanen und Cycloalkanen.

Daneben sind aber auch polare organische Lösungsmittel geeignet. Dies ist entgegen der Lehre von Gassman (J. Am. Chem. Soc., 1972, 94, 3891), wonach N-Chlor-Derivate in polaren Lösungsmittel weniger stabil sind. So kann die Reaktion im erfindungsgemäßen Verfahren vorzugsweise in einem Ester-Lösungsmittel, wie zum Beispiel C1-C6 Alkylacetat (beispielsweise Methylacetat, Ethylacetat, n-Propylacetat, iso-Propylacetat, 2-Methyl-Prop-1-ylacetat, n-Butylacetat, But-2-ylacetat, Pentylacetate, Hexylacetate und Cyclalkylacetate, C1-C6 Alkyl und Cycloalkylpropionate, C1-C6 Alkyl und Cycloalkyl -n-butyrate, -iso-butyrate, - pentanoate und -hexanoate und -cyclopentanoate und -cyclohexanoate) oder in Mischungen davon oder in Mischung mit anderen Lösungsmitteln, durchgeführt werden.

Das erfindungsgemäße Verfahren zur Herstellung von Verbindungen der Formel (4), basiert darauf, dass die beiden Verbindungen Anilin (Verbindung der Formel Q) und Thioether (Verbindung der Formel W) vorteilhaft vorgemischt werden können.

Die hieraus resultierende selektive und saubere Reaktion ist besonders überraschend und steht im Gegensatz zur Lehrmeinung, wonach Gassman demonstrierte, dass je nach Substituent eine N-Chlorierung oder eine S-Chlorierung optimal für die Reaktion ist (Gassman, J. Am. Chem. Soc., 1974, 96(17), 5512). Weiterhin würde man von einer Mischung, bestehend aus Anilin und Thioether, keine Selektivität bei einer Chlorierung erwarten, warum nach bisheriger Lehrmeinung nur stufenweise gearbeitet wurde.

Bis zu 1 Äquivalent, bevorzugt 0,5 bis 1,0 Äquivalente, besonders bevorzugt 0,7 bis 1,0 Äquivalente, insbesondere bevorzugt 0,8 bis 0,95 Äquivalente des Chlorierungsmittels werden zu einer Mischung eines Äquivalents des Thioethers (Verbindung der Formel W) und 2,0 bis 5,0 Äquivalente, bevorzugt 2,0 bis 3,0 Äquivalente, besonders bevorzugt 2,0 bis 2,5 Äquivalente des, Anilins (Verbindung der Formel Q) zugegeben.

Das Chlorierungsmittel kann vorzugsweise mit einem Lösungsmittel oder einem Lösungsmittelgemisch vorverdünnt werden und kann vorzugsweise vorgekühlt werden.

Eine besondere Ausführungsform der Erfindung ist, dass auch eine Teilmenge des Anilins (zwischen 1 und 99 Gew.%, bevorzugt zwischen 20 und 80 Gew.%, besonders bevorzugt 30 bis 70 Gew.% der Gesamtmenge an Anilin) getrennt vom, aber simultan mit dem Chlorierungsmittel, zugegeben werden.

Eine besondere Ausführungsform der Erfindung ist, dass bei dem erfindungsgemäßen Verfahren zur Herstellung von Verbindungen der Formel (4) auf die Zugabe eines zusätzlichen tertiären Amins verzichtet werden kann.

So wurde gefunden, dass ein Überschuss an den überraschenderweise elektronenarmen Anilinen als milde Base bei der Bildung von Produkt der Formel (4) fungiert. Dies ist überraschend gegenüber der Standard-Gassman-Reaktion, welche die Zugabe eines zusätzlichen tertiären Amins verwendet (vgl. Schemata 2 und 3: C = Tertiäre Amin-Base, z.B.: Triethylamin). Das Anilin ist offensichtlich in der Lage, die Umlagerung zum Produkt der Formel (4) zu katalysieren und daher auch HCl von einem Chlorsulfonium-Zwischenprodukt der Formel (3) zu eliminieren, welches nur zu Nebenreaktionen führen würde. Trotzdem und gerade deswegen ist es sehr überraschend, dass die Reaktion so durchgeführt werden kann. Gleichzeitig ist der Verzicht auf ein zusätzliches tertiäres Amin in dem erfindungsgemäßen Verfahren vorteilhaft, da auf eine spätere aufwendige Trennung von zurück gewonnenem Anilin und tertiärem Amin verzichtet werden kann.

Gegenstand der Erfindung ist auch das Verfahren zur Herstellung von Verbindungen der Formeln (7) und (8): 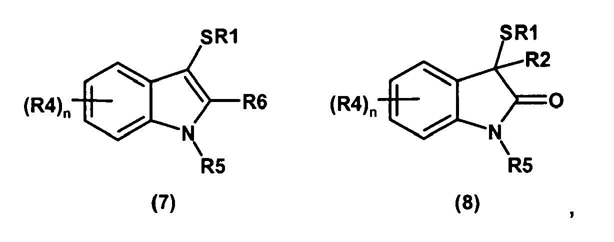 worin jeder der Reste
- R1: = C1-C6 Alkyl, substituiertes Alkyl, Aryl oder substituiertes Aryl, vorzugsweise C1-C4 Alkyl;
- R2: = H, C1-C6 Alkyl, substituiertes Alkyl, Aryl oder substituiertes Aryl;
- R4: = F, Cl, Br, I, CF₃, CN, NO₂, COX; wobei X = OR1, SR1, NR2R2', wobei R2' wie R2 oben definiert ist und gleich oder anders als R2 sein kann, vorzugsweise F, insbesondere 2-F;
- n: = 1 - 4, vorzugsweise 1 - 2 , insbesondere 1;
- R5: = H, C1-C6 Alkyl, substituiertes Alkyl, Aryl oder substituiertes Aryl;
- R6: = C1-C6 Alkyl, substituiertes Alkyl, Aryl oder substituiertes Aryl, vorzugsweise C1-C4 Alkyl oder OH; bedeutet,
wobei Verbindungen der Formel (4): 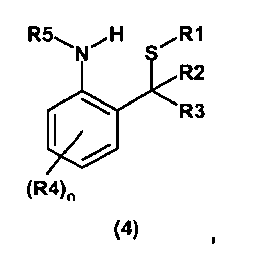 mit den oben genannten Definitionen der Reste R1, R2, R4 und R5 und wobei R3 eine elektronenziehende oder aktivierende Gruppe, wie -CO-R1; - CO-X, wobei X = OR1, SR1, NR2R2', wobei R2' wie R2 definiert ist und gleich oder anders als R2 sein kann; R2 und R2' können einen Ring bilden; SO(n')-R1, wobei n' ist 0, 1, or 2; -CN; -NO₂, Aryl or Heteroaryl; bedeutet,
mit oder ohne Isolierung der Verbindung der Formel (4), möglicherweise für Verbindungen der Formel (7) und vorzugsweise für Verbindungen der Formel (8), in Gegenwart eines Säure-Katalysators zu dem Indol der Formel (7) oder dem Oxindol der Formel (8) umgesetzt werden.

Im Falle von R3 = CO-R1 erhält man das Indol der Formel (7), während man im Falle von R3 = COX das Oxindol der Formel (8) oder das Indol der Formel (7) mit R6 = OH erhält.

Für den Säure-Katalysator im erfindungsgemäßen Verfahren können mineralische oder organische Säuren wie H⁺X⁻, wobei X⁻ = F⁻, Cl⁻, Br⁻, I⁻, HSO₄⁻, BF₄⁻, H₂PO₄⁻, SO₄²⁻, HPO₄²⁻, PO₄³⁻, R'SO₃⁻, R"HPO₃⁻, R"'PO₃²⁻, R""CO₂⁻ bedeutet, oder Mischungen davon, eingesetzt werden. Bevorzugt sind Mineralsäuren, insbesondere HCl, gasförmig oder in einem Lösungsmittel, vorzugsweise Wasser oder Alkohol. Der im erfindungsgemäßen Verfahren verwendete Säure-Katalysator wird in dem Fachmann hierfür gebräuchlichen Mengen eingesetzt.

Basierend auf dem Standard-Verfahren von Gassman et. al. (J. Am. Chem. Soc., 1974, 96(17), 5508) sind mehrere Oxindole oder ortho-substituierte Aniline synthetisiert worden. So wurde z.B. auf dem Weg der Synthese eines 7-Fluorisatins das Zwischenprodukt 7-Fluor-3-methylthio-oxindol synthetisiert (Wierenga et al., Tetrahedron Lett. 1983, 24, 2437). Weitere 7-Fluor-3-alkylthio-oxindole sowie deren Vorstufen sind im Stand der Technik nicht beschrieben.

Gegenstand der Erfindung sind daher auch Zwischenprodukte der Formel (4'), welches durch das oben beschriebene erfindungsgemäße Verfahren erhältlich sind und welche neu sind: 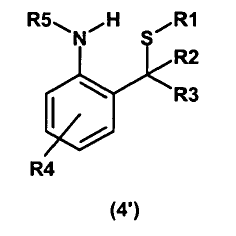 wobei
R1 = C2-C6 Alkyl, Benzyl;
R2 = H; R3 = CO₂R", wobei R" = C1-C6 Alkyl, Benzylester sein kann;
R4 = 2-F;
R5 = H, C1-C4 Alkyl;
bedeutet.
Bevorzugt sind Verbindungen der Formel (4'), wobei
R1 = Ethyl;
R2 = H;
R3 = CO₂Methyl, CO₂Ethyl;
R4 = 2-F;
R5 = H, Methyl, Ethyl;
bedeutet.

Gegenstand der Erfindung sind auch Endprodukte der Formel (7'), welche durch das oben beschriebene erfindungsgemäße Verfahren erhältlich sind und welche neu sind: 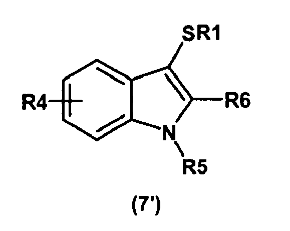 wobei
R1 = C2-C6 Alkyl, Benzyl;
R4 = 7-F;
R5 = H, C1-C4 Alkyl;
R6 = C1-C6 Alkyl, Benzyl oder OH sein kann; bedeutet.
Bevorzugt sind Verbindungen der Formel (7'), wobei R1 = Ethyl;
R4 = 7-F;
R5 = H, Methyl;
R6 = C1-C6 Alkyl, Benzyl oder OH sein kann;
bedeutet.

Gegenstand der Erfindung sind auch Endprodukte der Formel (8'), welche durch das oben beschriebene erfindungsgemäße Verfahren erhältlich sind und welche neu sind: 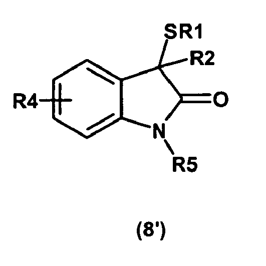 wobei
R1 = C2-C6 Alkyl, Benzyl;
R2 = H;
R4 = 7-F;
R5 = H, C1-C4 Alkyl;
bedeutet.
Bevorzugt sind Verbindungen der Formel (8'), wobei
R1 = Ethyl;
R2 = H;
R4 = 7-F;
R5 = H, Methyl;
bedeutet.

Gegenstand der Erfindung ist auch die Verwendung der erfindungsgemäß erhaltenen, neuen Verbindungen der Formeln (4'), (7') und (8') zur Herstellung von Weiterverarbeitungsprodukten und entsprechende Verfahren, dadurch gekennzeichnet, dass die Gruppe SR1 durch Wasserstoff ersetzt wird mit einem dem Fachmann bekannten Desulfonierungs-Verfahren.

Im Zusammenhang mit den in dieser Beschreibung verwendeten chemischen Begriffen gelten die für den Fachmann üblichen Definitionen, sofern nichts anderes spezifisch definiert ist. Die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C Atomen bzw. bei ungesättigten Gruppen mit 2 bis 6 C Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl.

Cycloalkyl bedeutet ein carbocyclisches, gesättigtes Ringsystem mit vorzugsweise 3 bis 8 C Atomen, z.B. Cyclopropyl, Cyclobutyl, Cyclopentyl oder Cyclohexyl.

Halogen bedeutet beispielsweise Fluor, Chlor, Brom oder Iod. Haloalkyl bedeutet durch Halogen, vorzugsweise durch Fluor, Chlor und/oder Brom, insbesondere durch Fluor und/oder Chlor, teilweise oder vollständig substituiertes Alkyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein heterocyclischer Rest oder Ring (Heterocyclyl) kann gesättigt, ungesättigt oder heteroaromatisch sein; er enthält vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono , bi oder polycyclisches aromatisches System, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Thiadiazolyl, Oxazolyl, Isoxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Oxazolinyl, Isoxazolinyl, Oxazolidinyl, Isoxazolidinyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie ein substituierter Alkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, Sulfamoyl, Mono und Dialkylaminosulfonyl, substituiertes Amino, wie Acylamino, Mono und Dialkylamino, und Alkylsulfinyl, Haloalkylsulfinyl, Alkylsulfonyl, Haloalkylsulfonyl und, im Falle cyclischer Reste, auch Alkyl und Haloalkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Phenyl, Phenoxy etc. eingeschlossen. Bei Resten mit C Atomen sind solche mit 1 bis 4 C Atomen, insbesondere 1 oder 2 C Atomen, bevorzugt. Bevorzugt sind in der Regel Substituenten aus der Gruppe Halogen, z.B. Fluor und Chlor, (C1-C4)Alkyl, vorzugsweise Methyl oder Ethyl, (C1-C4)Haloalkyl, vorzugsweise Trifluormethyl, (C1-C4)Alkoxy, vorzugsweise Methoxy oder Ethoxy, (C1-C4)Haloalkoxy, Nitro und Cyano. Besonders bevorzugt sind dabei die Substituenten Methyl und Fluor.

Von den Formeln (4'), (7') und (8') sind, soweit zutreffend, auch alle Stereoisomeren umfasst. Solche Verbindungen enthalten ein oder mehrere asymmetrische C-Atome, die in den allgemeinen Formeln nicht gesondert angegeben sind. Die durch ihre spezifische Raumform definierten möglichen Stereoisomeren, wie Enantiomere, Diastereomere, können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die erfindungsgemäß einzusetzenden Verbindungen der Formeln Q und W sind bekannt oder können analog allgemein bekannten Verfahren hergestellt werden.

Das erfindungsgemäße Verfahren zum Erhalt von Verbindungen der Formel (4) wird beispielsweise so durchgeführt, dass man Anilin (Verbindung der Formel Q) und Thioether (Verbindung der Formel W) im Lösungsmittel vorlegt und, vorzugsweise unter Schutzgas, herunterkühlt. Zu dieser gerührten Lösung wird eine Mischung aus Chlorierungsmittel und Lösungsmittel zugetropft. Die Reaktion wird anschließend erwärmt und mit einer wässrigen Säure verdünnt. Die Phasen werden getrennt und die organische Phase mit einer wässrigen Säure gewaschen. Die organische Phase wird anschließend getrocknet und im Falle, dass die Verbindung der Formel (4) ein Feststoff ist, *in vacuo* aufkonzentriert und möglicherweise ein Antilösungsmittel zugegeben und mehrere Stunden gerührt. Der Feststoff (Verbindung der Formel (4)) wird abfiltriert und mit dem Antilösungsmittel oder Lösungsmittelgemisch gewaschen. Im Falle, dass die Verbindung der Formel (4) ein Öl ist, wird die organische Phase *in vacuo* aufkonzentriert.

Das erfindungsgemäße Verfahren zum Erhalt von Verbindungen der Formeln (7) und (8) wird beispielsweise so durchgeführt, dass man Anilin (Verbindung der Formel Q) und Thioether (Verbindung der Formel W) im Lösungsmittel vorlegt und, vorzugsweise unter Schutzgas, herunterkühlt. Zu dieser gerührten Lösung wird eine Mischung aus Chlorierungsmittel und Lösungsmittel zugetropft. Die Reaktion wird anschließend erwärmt und mit einer wässrigen Säure verdünnt. Die Phasen werden getrennt und die organische Phase mit einer wässrigen Säure gewaschen. Die organische Phase wird anschließend, möglicherweise für Verbindungen der Formel (7) und vorzugsweise für Verbindungen der Formel (8), mit einem Säurekatalysator versetzt und mehrere Stunden gerührt. 90% des Lösungsmittels wird anschließend *in vacuo* entfernt und ein Antilösungsmittel zugegeben und mehrere Stunden gerührt. Der Feststoff (Verbindung der Formel (7) oder (8)) wird abfiltriert und mit dem Antilösungsmittel oder Lösungsmittelgemisch gewaschen.

Die folgenden Beispiele erläutern das erfindungsgemäße Verfahren näher, ohne das erfindungsgemäße Verfahren darauf zu beschränken. In den folgenden Beispielen beziehen sich Mengenangaben auf das Gewicht, sofern nichts anderes speziell definiert ist (in der Beschreibung wurde hierfür analog Gew.% = Gewichtsprozent verwendet). Für Maßeinheiten, physikalische Größen und ähnliches werden übliche Abkürzungen verwendet, beispielsweise h = Stunde(n), mpt. = Schmelzpunkt (Smp.), l = Liter, ml = Milliliter, g = Gramm, min = Minute(n), *in vacuo =* "im Vakuum" = unter reduziertem Druck, d. Th. = Prozent Ausbeute nach der Theorie.

### Beispiele

### Synthesebeispiel 1:

2-Chloranilin (20,4g) und Methylmercaptoessigsäure-methylester (8,36g) wurden in Chlorbenzol (77ml) vorgelegt und unter Schutzgas auf -30°C gekühlt. Zu dieser gerührten Lösung wurde eine Lösung von Sulfurylchlorid (8,45g) in Chlorbenzol (67ml) in 30 Minuten zugetropft. Die Reaktionsmischung wurde auf -5°C erwärmt und mit 0,4N Salzsäure (110ml) verdünnt. Die Phasen wurden getrennt und die organische Phase mit 0,4N Salzsäure gewaschen (4 x 110ml). Die organische Phase wurde dann mit 10%iger HCl in MeOH (2ml) versetzt und 16 Stunden gerührt. 90% des Lösungsmittels wurden *in vacuo* entfernt, Heptan (120ml) zugegeben und 4 Stunden gerührt. Der Feststoff wurde abfiltriert und mit Heptan (2 x 50ml) gewaschen. Man erhält 7-Chlor-3-methylthio-oxindole (9,36g 70% d. Th.). mpt.: 167-170°C.

### Synthesebeispiel 2:

2-Chloranilin (20,4g) und Methylmercaptoessigsäure-methylester (8,36g) wurden in Chlorbenzol (77ml) vorgelegt und unter Schutzgas auf -30°C gekühlt. Zu dieser gerührten Lösung wurde eine Lösung von Sulfurylchlorid (8,45g) in Chlorbenzol (67ml) in 30 Minuten zugetropft. Die Reaktionsmischung wurde auf -5°C erwärmt und mit 0,4N Salzsäure (110ml) verdünnt. Die Phasen wurden getrennt, die organische Phase mit 0,4N Salzsäure (4 x 110ml) und Wasser (50ml) gewaschen und über Natriumsulfat getrocknet. Die organische Phase wurde filtriert und *in vacuo* abdestilliert. Man erhält (2-Amino-3-chlophenyl)-(methylthio)essigsäure-methylester als braun-rotes Öl (10,9 g 71% d.Th.).

### Synthesebeispiel 3:

4-Chloranilin (20,4g) und Methylmercaptoessigsäure-methylester (8,36g) wurden in Essigsäure-n-butylester (77ml) vorgelegt und unter Schutzgas auf -30°C gekühlt. Zu dieser gerührten Lösung wurde eine Lösung von Sulfurylchlorid (8,45g) in Essigsäure-n-butylester (67ml) in 30 Minuten zugetropft. Die Reaktion wurde auf 10°C erwärmt und mit 0,4N Salzsäure (110ml) verdünnt. Die Phasen wurden getrennt und die organische Phase mit 0,4N Salzsäure gewaschen (2 x 110ml). Die organische Phase wurde dann mit 10%iger HCl in MeOH (2ml) versetzt und 16 Stunden gerührt. 90% des Lösungsmittels wurden *in vacuo* entfernt, Heptan (120ml) zugegeben und 4 Stunden gerührt. Der Feststoff wurde abfiltriert und mit Heptan (2 x 50ml) gewaschen. Man erhält 5-Chlor-3-methylthio-oxindole (7,89g 59% d. Th.). mpt.: 154-157°C.

### Synthesebeispiel 4:

2-Fluoranilin (17,8g) und Methylmercaptoessigsäure-methylester (8,36g) wurden in Essigsäure-n-butylester (77ml) vorgelegt und unter Schutzgas auf -30°C gekühlt. Zu dieser gerührten Lösung wurde eine Lösung von Sulfurylchlorid (8,45g) in Essigsäure-n-butylester (67ml) in 30 Minuten zugetropft. Die Reaktion wurde auf 10°C erwärmt und mit 0,4N Salzsäure (110ml) verdünnt. Die Phasen wurden getrennt und die organische Phase mit 0,4N Salzsäure gewaschen (1 x 110ml). Die organische Phase wurde dann mit 10%iger HCl in MeOH (2ml) versetzt und 16 Stunden gerührt. 90% des Lösungsmittels wurden *in vacuo* entfernt und Heptan (120ml) zugegeben und 4 Stunden gerührt. Der Feststoff wurde abfiltriert und mit Heptan (2 x 50ml) gewaschen. Man erhält 7-Fluor-3-methylthio-oxindole (9,17g 72% d. Th.). mpt.: 156-159°C.

### Synthesebeispiel 5:

2-Fluoranilin (17,8g) und Methylmercaptoessigsäure-methylester (8,36g) wurden in Essigsäure-n-butylester (77ml) vorgelegt und unter Schutzgas auf -30°C gekühlt. Zu dieser gerührten Lösung wurde eine Lösung von Sulfurylchlorid (8,45g) in Essigsäure-n-butylester (67ml) in 30 Minuten zugetropft. Die Reaktion wurde auf 10°C erwärmt und mit 0,4N Salzsäure (110ml) verdünnt. Die Phasen wurden getrennt, die organische Phase mit 0,4N Salzsäure (1 x 110ml) und Wasser (50ml) gewaschen und über Natriumsulfat getrocknet. Die organische Phase wurde filtriert und *in vacuo* abdestilliert. Man erhält (2-Amino-3-fluorphenyl)-(methylthio)essigsäure-methylester als braunes Öl (12,5 g 73% d.Th.).

## Patentansprüche

1. Verfahren zur Herstellung von Verbindungen der Formel (4): 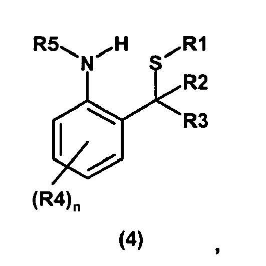 worin jeder der Reste
R1 = C1-C6 Alkyl, substituiertes Alkyl, Aryl oder substituiertes Aryl, vorzugsweise C1-C4 Alkyl;
R2 = H, C1-C6 Alkyl, substituiertes Alkyl, Aryl oder substituiertes Aryl;
R3 = elektronenziehende oder aktivierende Gruppe, wie -CO-R1; -CO-X, wobei X = OR1, SR1, NR2R2', wobei R2' wie R2 definiert ist und gleich oder anders als R2 sein kann; R2 und R2' können einen Ring bilden; SO(n')-R1, wobei n' ist 0, 1, or 2; -CN; -NO₂, Aryl or Heteroaryl;
R4 = F, Cl, Br, I, CF₃, CN, NO₂, COX; wobei X = OR1, SR1, NR2R2', wobei R2' wie R2 oben definiert ist und gleich oder anders als R2 sein kann, vorzugsweise F, insbesondere 2-F;
n = 1 - 4, vorzugsweise 1 - 2 , insbesondere 1;
R5 = H, C1-C6 Alkyl, substituiertes Alkyl, Aryl oder substituiertes Aryl;
bedeutet,
**dadurch gekennzeichnet, dass** man eine Mischung
eines Anilins (Verbindung der Formel Q): 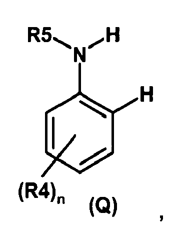 worin die Reste R4, n und R5 wie in Formel (4) definiert sind, mit einem Thioether (Verbindung der Formel W): 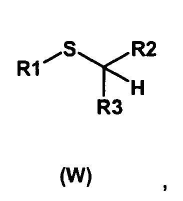 worin die Reste R1, R2 und R3 wie in Formel (4) definiert sind,
in Gegenwart eines Chlorierungsmittels und eines organischen Lösungsmittels bei einer Reaktionstemperatur im Bereich von über -65°C, vorzugsweise zwischen -60 und -10°C, insbesondere zwischen -50 und -20°C, zur Verbindung der Formel (4) umsetzt.

2. Verfahren nach Anspruch 1, **dadurch gekennzeichnet, dass** die Reaktion in Ester-Lösungsmitteln, vorzugsweise ausgewählt aus der Gruppe C1-C6 Alkylacetat (beispielsweise Methylacetat, Ethylacetat, n-Propylacetat, iso-Propylacetat, 2-Methyl-Prop-1-ylacetat, n-Butylacetat, But-2-ylacetat, Pentylacetate, Hexylacetate und Cyclalkylacetate, C1-C6 Alkyl und Cycloalkylpropionate, C1-C6 Alkyl und Cycloalkyl -n-butyrate, -iso-butyrate, -pentanoate und -hexanoate und -cyclopentanoate und -cyclohexanoate) oder in Mischungen davon oder in Mischung mit anderen Lösungsmitteln, durchgeführt wird.

3. Verfahren nach Anspruch 1 oder 2, **dadurch gekennzeichnet, dass** bis zu 1 Äquivalent, bevorzugt 0,5 bis 1,0 Äquivalente, besonders bevorzugt 0,7 bis 1,0 Äquivalente, insbesondere bevorzugt 0,8 bis 0,95 Äquivalente des Chlorierungsmittels zu einer Mischung eines Äquivalents des Thioethers (Verbindung der Formel W) und 2,0 bis 5,0 Äquivalente, bevorzugt 2,0 bis 3,0 Äquivalente, besonders bevorzugt 2,0 bis 2,5 Äquivalente des Anilins (Verbindung der Formel Q) zugegeben wird.

4. Verfahren nach einem der Ansprüche 1 bis 3, **dadurch gekennzeichnet, dass** auch eine Teilmenge des Anilins (zwischen 1 und 99 Gew.%, bevorzugt zwischen 20 und 80 Gew.%, besonders bevorzugt 30 bis 70 Gew.% der Gesamtmenge an Anilin) getrennt vom, aber simultan mit dem Chlorierungsmittel zugegeben wird.

5. Verfahren nach einem der Ansprüche 1 bis 4, **dadurch gekennzeichnet, dass** auf die Zugabe eines zusätzlichen tertiären Amins verzichtet wird.

6. Verfahren zur Herstellung von Verbindungen der Formeln (7) und (8): 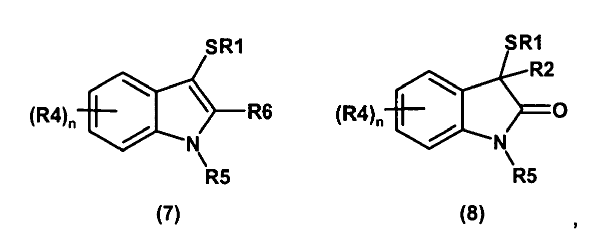 worin jeder der Reste
R1 = C1-C6 Alkyl, substituiertes Alkyl, Aryl oder substituiertes Aryl, vorzugsweise C1-C4 Alkyl;
R2 = H, C1-C6 Alkyl, substituiertes Alkyl, Aryl oder substituiertes Aryl;
R4 = F, CI, Br, I, CF₃, CN, NO₂, COX; wobei X = OR1, SR1, NR2R2', wobei R2' wie R2 oben definiert ist und gleich oder anders als R2 sein kann, vorzugsweise F, insbesondere 2-F;
n = 1 - 4, vorzugsweise 1 - 2 , insbesondere 1;
R5 = H, C1-C6 Alkyl, substituiertes Alkyl, Aryl oder substituiertes Aryl;
R6 = C1-C6 Alkyl, substituiertes Alkyl, Aryl oder substituiertes Aryl,
vorzugsweise C1-C4 Alkyl oder OH; bedeutet,
wobei Verbindungen der Formel (4): 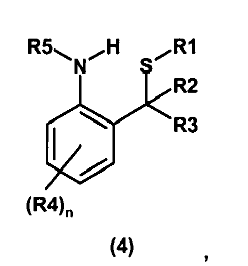 mit den oben genannten Definitionen der Reste R1, R2, R4, n und R5 und wobei R3 eine elektronenziehende oder aktivierende Gruppe, wie -CO-R1; - CO-X, wobei X = OR1, SR1, NR2R2', wobei R2' wie R2 definiert ist und gleich oder anders als R2 sein kann; R2 und R2' können einen Ring bilden; SO(n')-R1, wobei n' ist 0, 1, or 2; -CN; -NO₂, Aryl or Heteroaryl; bedeutet,
mit oder ohne Isolierung der Verbindung der Formel (4), möglicherweise für Verbindungen der Formel (7) und vorzugsweise für Verbindungen der Formel (8), in Gegenwart eines Säure-Katalysators zu dem Indol der Formel (7) oder dem Oxindol der Formel (8) umgesetzt werden.

7. Zwischenprodukte der Formel (4') 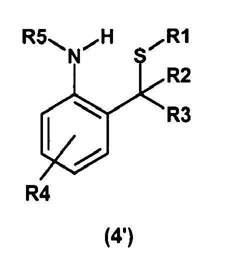 wobei
R1 = C2-C6 Alkyl, Benzyl;
R2 = H;
R3 = CO₂R", wobei R" = C1-C6 Alkyl, Benzylester sein kann; R4 = 2-F;
R5 = H, C1-C4 Alkyl;
bedeutet.

8. Endprodukte der Formel (7') 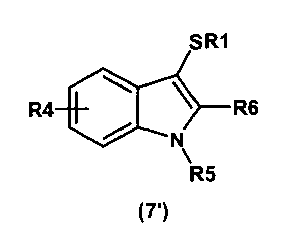 wobei
R1 = C2-C6 Alkyl, Benzyl;
R4 = 7-F;
R5 = H, C1-C4 Alkyl;
R6 = C1-C6 Alkyl, Benzyl oder OH sein kann; bedeutet.

9. Endprodukte der Formel (8') 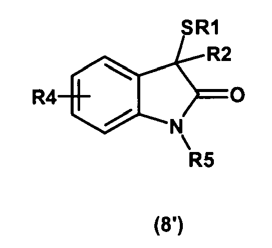 wobei R1 = C2-C6 Alkyl, Benzyl; R2 = H;
R4 = 7-F;
R5 = H, C1-C4 Alkyl; bedeutet.

10. Verwendung der Verbindungen nach einem oder mehreren der Ansprüche 7 bis 9 zur Herstellung von Weiterverarbeitungsprodukten und entsprechende Verfahren, dass die Gruppe SR1 durch Wasserstoff ersetzt wird mit einem dem Fachmann bekannten Desulfonierungs-Verfahren.
